# EUROPÄISCHE PATENTSCHRIFT

(11) **EP 0 473 965 B1**
(45) Veröffentlichungstag und Bekanntmachung des Hinweises auf die Patenterteilung: **28.02.1996**
(21) Anmeldenummer: 91113392.4
(22) Anmeldetag: 09.08.1991
(51) Int. Cl.: A61M 15/00, A61M 11/00

(54) **Austragvorrichtung für Medien**
Dispensing device for substances
Distributeur pour substances

(30) Priorität: 01.09.1990 DE 4027749
(43) Veröffentlichungstag der Anmeldung: 11.03.1992
(60) Teilanmeldung: 95111049.3
(73) Patentinhaber: Ing. Erich Pfeiffer GmbH & Co. KG, D-78315 Radolfzell (DE)
(72) Erfinder: Wolter, Michael, CH-8266 Steckborn (CH); Zuckschwerdt, Friedrich, W-7760 Radolfzell (DE)
(74) Vertreter: Patentanwälte Ruff, Beier, Schöndorf und Mütschele

(56) Entgegenhaltungen:
- WO-A-90/07351
- CH-A- 258 145
- DE-B- 1 016 198
- GB-A- 24 848
- US-A- 1 552 181

## Beschreibung

Die Erfindung betrifft eine Austragvorrichtung nach dem Oberbegriff des Patentanspruches 1. Sie kann für ein einziges Medium bzw. für gesondert gespeicherte, gleiche oder ungleiche fließfähige Medien von ggf. unterschiedlichem Aggregatzustand geeignet sein, wobei das Medium bevorzugt wenigstens teilweise rieselfähig, z.B. pulver- und/oder puderförmig, sein kann.

Zweckmäßig ist die Austragvorrichtung so ausgebildet, daß das Medium mit einem Gasstrom aus einer oder mehreren Medienkammern herausgefördert bzw. herausgerissen wird, wobei die jeweilige Medienkammer unmittelbar durch einen Medienspeicher gebildet sein kann.

Die Austragvorrichtung kann z.B. als Inhalator für pulverförmige Substanzen verwendet werden. Bei ihr kann z.B. eine das Pulver enthaltende Kapsel durch Zerstörung geöffnet und deren Inhalt mit der Atemluft der inhalierenden Person abgesaugt werden.

Die Austragvorrichtung kann auch so ausgebildet sein, daß flüssige oder pulverförmige Stoffe mittels eines Druckgasstromes zur Erzeugung eines Aerosols zerstäubt, verteilt und vermischt werden, jedoch muß hier die Austragvorrichtung an eine externe Druckgasquelle angeschlossen werden, was ihre Benutzbarkeit stark einschränkt.

Ist die Austragvorrichtung mit einer Luftpumpe kombiniert, so kann die Anordnung so vorgesehen sein, daß das Medium mit dem Druckluftstrom nicht aus einer Kammer herausgefördert werden kann, sondern daß der Druckluftstrom lediglich getrennt von dem Medium einer Austragdüse zugeführt und dort erst mit dem Medium gemischt wird, so daß das Medium zunächst unabhängig vom Luftstrom über eine gesonderte Pumpe bis zur Austragdüse gefördert wird.

Durch die GB-A-24 848 ist eine Austragvorrichtung bekannt geworden, bei welcher die Gas-Mündung gleich ausgerichtet ist wie die Strömung des Mediums im Auslaßkanal.

Die CH-A-258 145 zeigt eine Austragvorrichtung, bei welcher in dem Bereich, in welchem ein Luftstrom und das Medium aufeinander treffen, gleiche Strömungsrichtungen von Luft und Medium vorgesehen sind.

Die DE-A-1 016 198 zeigt eine Austragvorrichtung, bei welcher das Medium dadurch vom Luftstrom mitgenommen wird, daß dieser an einem Ende in eine Vorlagekammer eintritt und am anderen Ende mit dem Medium austritt.

Der Erfindung liegt die Aufgabe zugrunde, eine Austragvorrichtung der genannten Art zu schaffen, bei welcher Nachteile bekannter bzw. der beschriebenen Ausbildungen vermieden sind und die insbesondere unabhängig von äußeren Druckgasquellen das wirksame Herausfördern des Mediums aus einer speicherartigen Medienkammer gewährleisten soll.

Zur Lösung dieser Aufgabe sind die Merkmale des Patentanspruches 1 geeignet. Die Austragvorrichtung soll eine handbetätigbare Vorrichtung zur Erzeugung von Druckgas aufweisen bzw. an eine solche Vorrichtung so anschließbar sein, daß das Druckgas wenigstens teilweise mittelbar und/oder unmittelbar, nämlich während der Betätigung, einer Zone zugeführt werden kann, an welcher es das zunächst nach Art einer Vorlage ruhende Medium aufnimmt, mitreißt und nach außen fördert. Die Austragvorrichtung ist dadurch jederzeit einsetzbar, sie kann sehr kompakt ausgebildet werden und sie ermöglicht die aerosolartige Ausbringung unterschiedlichster Stoffe verschiedener Aggregatzustände, insbesondere von pharmazeutischen, kosmetischen und ähnlichen Substanzen.

Zweckmäßig weist die Vorrichtung einen gegen eine bzw. entlang einer Vorlage für das Medium gerichteten Injektor bzw. einen Wirbelkanal auf, in dessen Bereich durch Querschnittsverengung verhältnismäßig große Strömungsgeschwindigkeiten und Turbulenzen des Gasstromes erzielt werden, so daß das Medium feinstverteilt und vermischt mitgerissen wird. Eine Druckgasmündung soll dabei etwa entgegen Strömungsrichtung aus diesem Bereich gerichtet sein.

Vorteilhaft ist eine Dosiereinrichtung für das Medium vorgesehen, so daß je manueller Betätigung nur eine ganz bestimmte Menge an Medium abgegeben und ausgetragen wird. Die Dosiereinrichtung ist zweckmäßig über eine Fließstrecke unmittelbar an einen Medienspeicher angeschlossen und kann außer durch Gewichtskraft auch dadurch in eine Dosierkammer gefördert werden, daß im entsprechenden Bereich durch den Gasstrom ein geringer Unterdruck erzeugt wird. Die Dosierkammer kann nach Füllung wegabhängig vom Betätigungsweg der Dosiereinrichtung eingangsseitig geschlossen werden, während zwischen der Dosierkammer bzw. der Medienvorlage und der ins Freie bzw. in ein Mundstück führenden Austragöffnung keinerlei Ventil erforderlich ist.

Durch die erfindungsgemäße Ausbildung bedarf es nur einer einzigen Pumpe, nämlich derjenigen für die Druckgaserzeugung, wobei jedoch auch mehrere Pumpen in Serie und/oder parallelgeschaltet vorgesehen sein können.

Obwohl andere Betätigungsbewegungen denkbar sind, ist die Austragvorrichtung zweckmäßig ausschließlich durch eine etwa lineare Hubbewegung zu betätigen, wobei ihre voneinander abgekehrten äußeren Endflächen, die durch zwei teleskopartig ineinandergreifende Bauteile gebildet sind, die Handhaben bilden. Einer der Bauteile kann dabei einen wenigstens teilweise freiliegenden und über eine verschließbare Öffnung nachfüllbaren Medienspeicher bilden, welcher zweckmäßig im wesentlichen lagestarr oder axial über eine Steuerstrecke beweglich einen Pumpkolben trägt, der in den anderen, ansonsten die Pumpenkammer begrenzenden Bauteil eingreift. Beide Bauteile sind in einfacher Weise durch Schnappverbindungen aneinander befestigt, so daß sich ein sehr einfacher Aufbau ergibt.

Diese und weitere Merkmale gehen außer aus den Ansprüchen auch aus der Beschreibung und der Zeichnung hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können. Ein Ausführungsbeispiel der Erfindung ist in der Zeichnung dargestellt und wird im folgenden näher erläutert. In der Zeichnung zeigt :

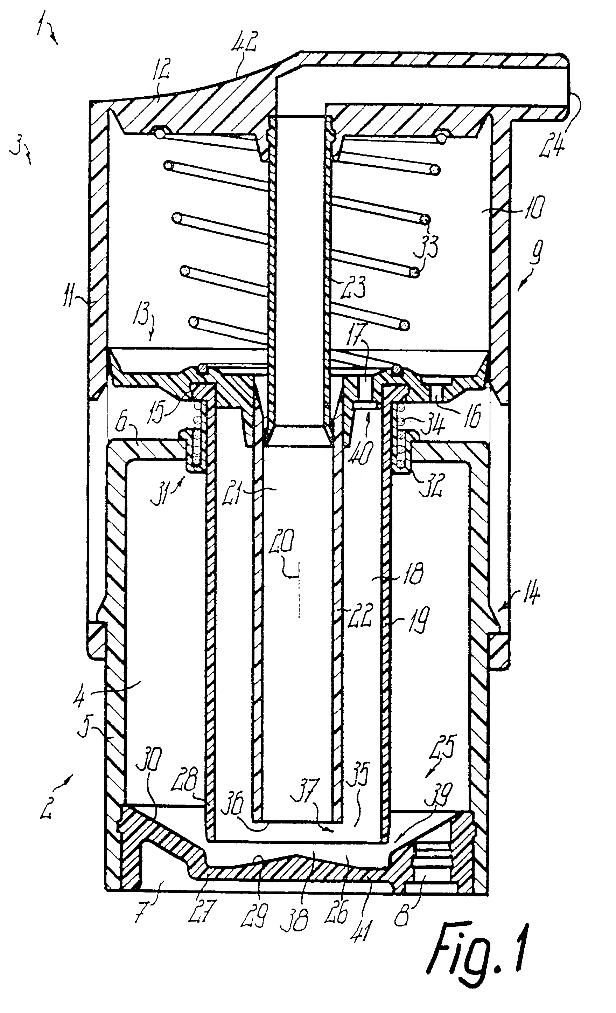

- Fig. 1: eine erfindungsgemäße Austragvorrichtung im Axialschnitt.

Die Austragvorrichtung 1 ist aus nur zwei vormontierten Baugruppen 2, 3 durch eine Steckverbindung zusammengesetzt und weist einen Medienspeicher 4 auf, aus dem je Betätigungshub eine Teilmenge ausgebracht wird. Eine untere Baugruppe 2 bildet mit ihrem äußeren Mantel 5 die äußere Begrenzung des Medienspeichers 4 und des zugehörigen Längsabschnittes der Austragvorrichtung. Der Mantel 5 bildet mit einer einteiligen, ringscheibenförmigen Stirnwand 6 an einem Ende und einem im wesentlichen vollständig versenkt eingesetzten Deckel 7 am anderen Ende einen im wesentlichen formstabilen Hohlkörper. Im eingeschnappten Deckel 5 ist eine exzentrische, mit einem Stopfen zu verschließende Füllöffnung 8 für den Medienspeicher 4 vorgesehen.

Die beiden Baugruppen 2, 3 bilden die beiden gegeneinander über einen Pumphub verschiebbar aneinander gelagerten Bestandteile einer Pumpe 9, deren Pumpenkammer 10 von den einander gegenüberliegenden Enden der beiden Baugruppen 2, 3 und einem äußeren Mantel 11 der Baugruppe 3 begrenzt ist, in dessen offenes Ende die Baugruppe 2 eingeschoben ist. Der Medienspeicher 4 und die Pumpenkammer 10 sind im Querschnitt ringförmig, wobei der lichte Querschnitt und die Außenweite der Pumpenkammer 10 größer als diejenige der Medienkammer 4 sind. Der Mantel 11 ist einteilig mit einer das andere Ende der Austragvorrichtung 1 bildenden Stirnwand 12 ausgebildet und nimmt als Kolbenlaufbahn einen dieser gegenüberliegenden, ringförmigen Pumpkolben 13 verschiebbar auf, welcher an der Baugruppe 2 gelagert ist und unmittelbar benachbart zur Stirnwand 6 liegt.

Die beiden Baugruppen 2, 3 sind durch mindestens eine Schnappverbindung 14 gegeneinander axial und gegen Verdrehung gesichert. Jede Schnappverbindung 14 weist am Außenumfang des Mantels 5 einen vorspringenden Schnappnocken und für dessen Eingriff im Mantel 11 einen Axialschlitz auf, wobei sich an die Axialschlitze die Kolbenlaufbahn bis zur Stirnwand 12 anschließt. Der, ebenfalls über eine Schnappverbindung 15 befestigte, napfförmig mit seinem Rand eine Dichtlippe bildende Pumpkolben 13 weist radial außerhalb seiner Schnappbefestigung im Boden mindestens einen Gaseintritt 16 für die Pumpenkammer 10 auf, der über die Axialschlitze ansaugen kann und mit einem nicht näher dargestellten, z.B. federfreien Rückschlagventil versehen ist. Innerhalb der Schnappverbindung 15 ist im Boden des Pumpkolbens 13 mindestens ein diesen ebenfalls durchsetzender Gasaustritt 17 vorgesehen, über welchen beim Pumphub die Luft aus der Druckkammer 10 in einen ringförmigen Strömungskanal 18 nach unten gedrückt wird. Der Strömungskanal 18 ist von einem innerhalb des Medienspeichers 4 liegenden und diesen am Innenumfang begrenzenden Kanalmantel 19 begrenzt, der die Stirnwand 6 durchsetzt und an dessen zugehörigem Ende der Pumpkolben 13 mit der Schnappverbindung 15 befestigt ist. Im wesentlichen alle genannten Bauteile liegen in einer zentralen Achse 20 der Austragvorrichtung 1.

Innerhalb des Strömungskanales 18 liegt berührungsfrei ein engerer Auslaßkanal 21, der von zwei mit den Baugruppen 2, 3 gegeneinander teleskopartig axial verschiebbaren Kanalteilen 22, 23 begrenzt ist. Ein Kanalteil 22, der den ringförmigen Strömungskanal 18 am Innenumfang begrenzt, ist mit einem Ende radial innerhalb des Gasaustrittes 17 in einer zentralen Öffnung des Pumpkolbens 13 befestigt und ragt wie der Kanalmantel 19 vom Pumpkolben bzw. von der Stirnwand 6 frei in den Medienspeicher 4. Der andere, mit einer Dichtlippe am Innenumfang des Kanalteiles 22 geführte Kanalteil 23 ist mit seinem entsprechenden Ende durch eine Schnappverbindung an der Stirnwand 12 befestigt und führt über einen in dieser liegenden Endabschnitt des Auslaßkanales zu einer Auslaßöffnung 24, die in der Achse 20 oder quer zu dieser gerichtet vorgesehen sein kann.

Zur Zumessung einer mit einem Pumphub auszubringenden Mediencharge ist eine Dosiereinrichtung 25 im Bodenbereich des Medienspeichers 4 vorgesehen, die eine in Abhängigkeit vom Pumphub zu öffnende und zu schließende Dosierkammer 26 aus zwei Kammerteilen 27, 28 aufweist. Der eine Kammerteil 27 ist im wesentlichen napfförmig und durch die zugehörig Stirnwand bzw. den Deckel 7 des Medienspeichers 4 gebildet. Der andere Kammerteil 28 ist durch den zugehörigen Endabschnitt des Kanalmantels 19 gebildet, der bis zum Anschlag an der Bodenfläche 29 eng angepaßt in den Mantel des Kammerteiles 27 einfahrbar ist und in Ausgangsstellung mit dem Kammerteil 27 eine ringschlitzförmige Übertrittsöffnung begrenzt. An den Mantel bzw. die Bodenfläche des Kammerteiles 27 schließt radial nach außen ein stumpfwinklig konischer Schütt-Trichter 30 an, dessen radial innerer Bereich die eine Begrenzung der Übertrittsöffnung bildet und über welchen das Medium aus dem Medienspeicher 4 auf die Bodenfläche 29 fließen kann. Die Bodenfläche 29 ist zweckmäßig zur Mitte hin erhöht, so daß das Medium auf ihr in einer ringförmigen Vorlage zu liegen kommt, die etwa deckungsgleich mit dem Strömungskanal 18 ist.

Der Kammerteil 28 ist mit einer Lagerung 31 für den Kanalmantel 19 axial zwischen der beschriebenen Schließstellung für die Übertrittsöffnung und der Ausgangsstellung verschiebbar an der Stirnwand 6 gelagert. Ein Stülpmantel 32 ist mit seinem radial äußeren Mantelabschnitt in der Durchtrittsöffnung der Stirnwand 6 und mit seinem radial inneren Mantelabschnitt am Außenumfang des dem Pumpkolben 13 zugehörigen Endes des Kanalmantels 19 befestigt, wobei die beiden Mantelabschnitte an ihren vom Pumpkolben 13 abgekehrten und innerhalb des Medienspeichers 4 liegenden Enden über einen Ringabschnitt ineinander übergehen. Zwischen den beiden Mantelabschnitten ist auf dem Außenumfang des Kanalabschnittes 19 eine Stützfeder angeordnet, die sich mit einem Ende am Ringabschnitt des Stülpmantels 32 abstützen und ggf. auch als Rückstellfeder 34 zur Rückstellung des Kammerteiles 28 in die Ausgangsstellung vorgesehen sein kann. Dieser, mit ihrem anderen Ende auf den Pumpkolben 13 axial wirkenden Rückstellfeder 34 wirkt eine stärkere Rückstellfeder 33 entgegen, die um den Kanalteil 23 in der Pumpenkammer 10 vorgespannt zwischen der Stirnwand 12 und dem Pumpkolben 13 angeordnet ist. Die auf den Kammerteil 28 wirkende Rückstellkraft ist mindestens so groß, daß sie bei Ausgangsstellung der Rückstellfeder 33 die Öffnung der Dosiereinrichtung 25 zuverlässig zuläßt. Je nach Abstimmung der beiden gegeneinander wirkenden Rückstellkräfte kann die Schließung der Dosiereinrichtung 25 bereits am Anfang des Pumphubes oder erst, z.B. druckabhängig von der Pumpe 9, nach einem ersten Teilweg des Pumphubes erfolgen.

Das untere Ende des Strömungskanales 18 bildet eine ringförmig gegen die Bodenfläche 29 gerichtete Mündung 35, die am Außenumfang vom Kammerteil 28, an einer Stirnseite von der vertieften Ringzone der Bodenfläche 29 und am Innenumfang vom zugehörigen Ende des Kanalteiles 22 begrenzt ist, das bei geschlossener Dosiereinrichtung 25 mit Spaltabstand dem kegelstumpfförmigen zentralen Bereich der Bodenfläche 29 gegenüberliegt. Dieses Ende des Kanalteiles 22 bildet gleichzeitig einen zentralen Ausgang 36 für das aus der Vorlage- bzw. Dosierkammer 26 durch den Auslaßkanal 21 herauszufördernde Medium bzw. den Eingang des Auslaßkanales 29. Durch die beschriebene Ausbildung ist im Bereich der Ring-Mündung 35, die in Pumpstellung gegenüber den Querschnitten des Ringkanales 18 wesentlich verengt ist, ein Injektor 37 mit einer Umlenkung des Gasstromes an der Bodenfläche 29 um etwa 180° sowie im wesentlichen durch den Mündungsbereich, die geschlossene Dosierkammer und den Ausgangsbereich eine Wirbelkammer 38 zur Aufwirbelung des Mediums gebildet.

Die Austragvorrichtung 1 arbeitet zweckmäßig nach folgendem Verfahren:
Die voneinander abgekehrten, durch den Deckel 7 und die Stirnwand 12 gebildeten Stirnflächen der Austragvorrichtung 1 dienen als Druck-Handhaben 41, 42 zur einhändigen, manuellen Betätigung der Austragvorrichtung. Die Austragvorrichtung kann dabei verhältnismäßig kleine Abmessungen in der Größenordnung von weniger als 50 mm Durchmesser und weniger als 80 mm Länge haben. Beim manuellen Zusammendrücken der beiden Baugruppen 2, 3 wird zunächst der Zylinder-Mantel 11 entgegen der Kraft der Rückstellfeder 33 gegenüber dem Pumpkolben 13 verschoben, so daß sich in der Pumpenkammer 10 ein Druck aufbaut. Je nachdem, ob dem ringförmig über den Strömungskanal 18 verteilten Gasaustritt 17 nur ein federfreies Rückschlagventil oder ein vorgespanntes Überdruckventil als Steuer-Ventil 40 zugeordnet ist, wird sofort oder erst nach Aufbau eines entsprechenden Überdruckes Druckluft in den Strömungskanal 18 gefördert. Vorher ist durch die ohnehin auftretenden Handhabungsbewegungen über den Trichter 30 aus dem Medienspeicher 14 Medium in die napfförmige Aufnahme des Kammerteiles 27 nachgerieselt. Nach Erreichen eines ersten Teilweges des Pumphubes wird der Kammerteil 28 über die Rückstellfeder 33 in die Schließstellung mitgenommen. Die währenddessen geförderte Luft bläst dabei, während die Übertrittsöffnung enger wird, den Sitz für den Kammerteil 28 von Medium im wesentlichen frei, bis die Übertrittsöffnung geschlossen und der Kammerteil 28 anschlagbegrenzt festgelegt ist.

Die weiterhin geförderte Luft reißt dann das auf der Bodenfläche 29 liegende Medium unter Verwirbelung und Umlenkung mit in den Ausgang 36, durch den Auslaßkanal 21 und nach außen durch die Auslaßöffnung 24. Gegen Ende des Pumphubes kann die Kraft der Rückstellfeder 33 so stark ansteigen, daß der Boden des Kammerteiles 27 unter dem Druck der manuell an ihm angreifenden Betätigungskraft elastisch geringfügig verformt, nämlich gegen den Ausgang 36 bewegt wird, so daß die Mündung 35, ohne zu schließen, kontinuierlich enger und die Strömungsgeschwindigkeit entsprechend höher wird. Dadurch ist eine sehr wirksame vollständige Entleerung der Dosierkammer gewährleistet. Wird danach die Austragvorrichtung 1 freigegeben, so kehrt zunächst die Pumpe 9 unter Schließung des Ventiles 40 zu ihrer Ausgangslage zurück, wonach auch die Dosierkammer 26 wieder öffnet, so daß sofort Medium nachfließen kann. Ggf. bis auf die Federn ist zweckmäßig mindestens einer bzw. sind im wesentlichen alle beschriebenen Bauteile aus Kunststoff hergestellt.

Die Pumpe kann auch an dem von der Auslaßöffnung abgekehrten Ende des Medienspeichers liegen. Die Pumpenkammer braucht dadurch von keinem Kanal durchsetzt zu sein. Sie weist dann in der Bodenwand des Pumpkolbens einen Gasaustritt auf, der in das eine Ende des im wesentlichen geradlinigen Strömungskanales mündet, dessen anderes Ende die Auslaßöffnung bildet. Das Ventil weist z.B. einen kugelförmigen, wenigstens teilweise innerhalb des zugehörigen Endes des Strömungskanales liegenden Ventilkörper auf, der mit einer innerhalb des Strömungskanales liegenden Ventilfeder zur Schließstellung federbelastet ist.

Der Kolben kann auch lagestarr über eine Schnappverbindung unmittelbar am zugehörigen Ende des Gehäuses des Medienspeichers befestigt sein. Die den Schnappverbindungen 14, 15 entsprechenden Schnappverbindungen können an gesonderten, hülsenartig ineinanderliegenden Mantelabschnitten des Speichergehäuses vorgesehen sein, dessen Speicherraum zweckmäßig zum Gasaustritt hin trichterartig verjüngt ist. Der Kanalmantel 19 kann auch den zum Nachfüllen abnehmbaren Deckel 7 durchsetzen, wenn dieser an dem zur Auslaßöffnung 24 benachbarten Ende des Medienspeichers 4 liegt. Die Pumpenkammer 10 kann an dem davon abgekehrten Ende mit einem entsprechenden, eingeschnappten Deckel verschlossen sein, der den ventilgesteuerten Gaseintritt 16 aufweisen kann.

## Patentansprüche

1. Austragvorrichtung für fließfähige flüssige bzw. pulverförmige Medien, die mit einem Gasstrom, insbesondere in mehreren aufeinanderfolgenden Chargen mit einer handbetätigbaren Druckgasquelle zur Erzeugung des Gasstromes, aus einer Medienkammer herauszufördern sind, mit Mitteln zur Abförderung des Mediums aus einer das Medium aufnehmenden Vorlage (29), wobei die Abfördermittel mindestens eine Gas-Strommündung (35) für den Austritt des das Medium von der Vorlage (29) aufnehmenden sowie von dieser heraus in wenigstens eine, eine Strömungsrichtung bestimmende Eingangsöffnung (36) eines Auslaßkanales (21) abfördernden Gasstromes aufweisen, dadurch gekennzeichnet, daß die Gasstrom-Mündung (35) etwa entgegen der Strömungsrichtung der Eingangsöffnung (36) ausgerichtet ist.

2. Austragvorrichtung nach Anspruch 1, dadurch gekennzeichnet, daß die Druckgasquelle wenigstens eine Pumpe (9) mit manuell veränderbarer Pumpkammer (10), insbesondere eine axial unmittelbar an einen Medienspeicher (4) anschließende Schubkolbenpumpe mit Pumpkolben (13) aufweist, deren Pumpenkammer (10) vorzugsweise eine Weite hat, die mindestens so groß wie die Weite des Medienspeichers (4) und/oder der übrigen Austragvorrichtung (1) ist.

3. Austragvorrichtung nach Anspruch 1 oder 2, dadurch gekennzeichnet, daß mindestens ein Strömungskanal (18) der Druckgasquelle (9) einen Medienspeicher (4), insbesondere im wesentlichen koaxial, durchsetzt und/oder daß der Medienspeicher (4) im Querschnitt im wesentlichen ringförmig ausgebildet ist.

4. Austragvorrichtung nach einem der vorhergehenden Ansprüche, dadurch gekennzeichnet, daß die Druckgasquelle (9) über mindestens einen Injektor (37) für die Mitnahme des Mediums bzw. über wenigstens eine Wirbelkammer (38) für das Medium an eine Auslaßöffnung (24) angeschlossen ist, wobei vorzugsweise mindestens eine Gasstrom-Mündung (36) als Strahldüse (35) in die Wirbelkammer (38) mündet und/oder daß mindestens ein Injektor (37) im Bereich des Anfanges eines insbesondere aufsteigenden Auslaßkanales (21) vorgesehen ist.

5. Austragvorrichtung nach einem der vorhergehenden Ansprüche, dadurch gekennzeichnet, daß ein Strömungskanal (18) mit wenigstens einer über seinen Umfang verteilten Medien-Eintrittsöffnung versehen ist, der vorzugsweise ein gesteuertes Ventil (40) für den Strömungskanal (18) vorgeschaltet und die insbesondere als Durchbruch in einem den Strömungskanal und die Medienkammer, trennenden Kanalmantel vorgesehen ist.

6. Austragvorrichtung nach einem der vorhergehenden Ansprüche, dadurch gekennzeichnet, daß eine Bodenfläche (29) als aus der Medienkammer (4) zu befüllende Vorlagefläche der Vorlage für Medium vorgesehen ist, gegen die vorzugsweise die als Druckgas-Mündung vorgesehene Gasstrom-Mündung (35) gerichtet ist und der im Abstand die Eingangsöffnung (36) des Auslaßkanales (21) gegenüberliegt, wobei vorzugsweise die Eingangsöffnung (36) ringförmig von der Gasstrom-Mündung (35) umgeben ist.

7. Austragvorrichtung nach einem der vorhergehenden Ansprüche, dadurch gekennzeichnet, daß eine Dosiereinrichtung (25) für das Medium vorgesehen ist, die vorzugsweise mit einer, zwei gegeneinander zwischen einer Füll- und einer Entleerstellung bewegbare Kammerteile (27, 28) aufweisenden, Dosierkammer (26) versehen und insbesondere im Bodenbereich der Medienkammer (4) angeordnet ist und daß vorzugsweise ein unterer Kammerteil (27) napfförmig und insbesondere am Rand von einem Schütt-Trichter (30) begrenzt bzw. ein oberer Kammerteil (28) insbesondere durch einen äußeren Mantelteil eines im Querschnitt ringförmigen oder einen Auslaßkanal (21) umgebenden Druckgas-Strömungskanales (18) gebildet ist, der insbesondere eng in den Mantel des unteren Kammerteiles (27) einfahrbar ist, wobei vorzugsweise die Dosierkammer (26) mit einer Steuerung, insbesondere weg- bzw. druckabhängig, verzögert gegenüber der Druckgasförderung schließbar und insbesondere ein bewegbarer Kammerteil (28) an einem dem Druckgas ausgesetzten Kolben, wie an dem Pumpkolben (13), angeordnet ist.

8. Austragvorrichtung nach einem der vorhergehenden Ansprüche, dadurch gekennzeichnet, daß ein bewegbarer Kammerteil (28) der Medienkammer (4) an einem einem weiteren Kammerteil (27) gegenüberliegenden Ende durchsetzt und vorzugsweise an einer zugehörigen Endwand (6) mit einem Stülpmantel (32) entgegen Federkraft etwa axial bewegbar gelagert ist, wobei insbesondere auf den bewegbaren Kammerteil (28) in entgegengesetzten Richtungen unterschiedlich starke Federn (33, 34) wirken.

9. Austragvorrichtung nach einem der vorhergehenden Ansprüche, dadurch gekennzeichnet, daß zur Medienförderung nur eine einzige Pumpe (9) vorgesehen ist, deren Pumpkolben (13) und/oder deren Pumpenzylinder vorzugsweise an der Medienkammer (4) mit mindestens einer Schnappverbindung (14, 15) angeordnet bzw. gelagert ist.

10. Austragvorrichtung nach einem der vorhergehenden Ansprüche, dadurch gekennzeichnet, daß ein Strömungskanal der Druckgasquelle (9), insbesondere der Auslaßkanal (21), durch einen Teleskopkanal gebildet ist, der vorzugsweise die Pumpenkammer (10) durchsetzt und/oder von dessen Kanalteilen (22, 23) einer an einem Pumpkolben (13) und einer an einer von zwei gegeneinander bewegbaren, durch voneinander abgekehrte Endwandungen der Medienkammer (4) und der Pumpenkammer (10) gebildeten Handhaben (41, 42) angeordnet ist.

## Claims

1. Discharge apparatus for flowable, liquid or pulverulent media, which can be delivered out of a medium chamber with a gas flow, particularly in several successive charges with a manually operable compressed gas source for producing the gas flow, with means for delivering the medium from a receiver (29) receiving said medium, the delivery means having at least one gas flow opening (35) for the exit of the gas flow receiving the medium from the receiver (29) and for delivering the same in at least one inlet opening (36) of an outlet channel (21) determining a flow direction, characterized in that the gas flow opening (35) is oriented roughly counter to the flow direction of the inlet opening (36).

2. Discharge apparatus according to claim 1, characterized in that the compressed gas device has a pump (9) with a manually variable pump chamber (10), particularly a thrust piston pump with pump piston (13) directly axially connectable to a medium reservoir (4) and whose pump chamber (10) preferably has a width which is at least as large as the width of the medium reservoir (4) and/or the remaining discharge apparatus (1).

3. Discharge apparatus according to claims 1 or 2, characterized in that at least one flow channel (18) of the compressed gas device (9) traverses a medium reservoir (4), particularly substantially coaxially and/or the medium reservoir (4) is cross-sectionally substantially circular.

4. Discharge apparatus according to any one of the preceding claims, characterized in that the compressed gas device (9) is connected by means of at least one injector (37) for entraining the medium and/or at least one turbulence chamber (38) for the medium to an outlet opening (24) and preferably at least one nozzle (35) issues into the turbulence chamber (38) and/or at least one injector (37) is provided in the vicinity of the start of a rising outlet channel (21).

5. Discharge apparatus according to any one of the preceding claims, characterized in that there is a flow channel (18) with at least one medium inlet distributed over a circumference upstream of which is preferably connected a controlled valve (40) for the flow channel (18) and which is in particular provided as an opening in a channel jacket separating the flow channel and the medium chamber.

6. Discharge apparatus according to any one of the preceding claims, characterized in that a bottom surface (29) is provided as a medium storage surface to be filled from the medium chamber (4), is directed against a compressed gas opening (35) and faces an inlet opening (36) of an outlet channel (21), the inlet opening (36) preferably being circularly surrounded by the compressed gas opening (35).

7. Discharge apparatus according to any one of the preceding claims, characterized in that a medium dosing mechanism (25) is provided, which preferably has a dosing chamber (26) with two chamber parts (27, 28) movable against one another between a filling and an emptying position and which is in particular located in the bottom region of the medium chamber (4) and that preferably a lower chamber part (27) is bounded in cup-shaped manner and in particular on the edge by a discharge hopper (30) and wherein an upper chamber part (28) is in particular formed by an outer jacket part of a cross-sectionally circular compressed gas flow channel (18) and/or surrounding an outlet channel (21) and which preferably is closely insertable into the jacket of the lower chamber part (27), and the dosing chamber (26) can preferably be closed by a control, particularly in path and/or pressure-dependent manner and delayed with respect to compressed air delivery and preferably a movable chamber part (28) is located on a piston exposed to the compressed gas and in particular on the pump piston (13).

8. Discharge apparatus according to any one of the preceding claims, characterized in that the movable chamber part (28) traverses the medium chamber (4) at an end opposite to the other chamber part (27) and is preferably axially movably mounted counter to spring tension on an associated end wall (6) with an inverted jacket (32) and in particular varyingly strong springs (33, 34) act in opposite directions on the movable chamber part (28).

9. Discharge apparatus according to any one of the preceding claims, characterized in that only one pump (9) is provided for medium delivery and its pump piston (13) and/or its pump cylinder is preferably arranged or mounted on the medium chamber (4) with at least one snap connection (14, 15).

10. Discharge apparatus according to any one of the preceding claims, characterized in that a flow channel of the compressed air device (9), particularly the outlet channel (21), is formed by a telescopic channel, which preferably traverses the pump chamber (10) and/or of whose channel parts (22, 23) one is located on a pump piston (13) and one on one of two handles (41, 42) movable against one another and formed by remote end walls of the medium chamber (4) and the pump chamber (10).

## Revendications

1. Distributeur pour substances capables de couler, liquides ou pulvérulentes, qui sont à transporter hors d'une chambre à substance avec un flux gazeux, en particulier en plusieurs charges successives, à l'aide d'une source de gaz sous pression pouvant être actionnée manuellement pour produire le flux gazeux, comprenant des moyens pour évacuer la substance d'un espace d'alimentation ou de prélèvement (29) recevant la substance, les moyens d'évacuation comportant au moins une embouchure de flux gazeux (35) pour la sortie du flux gazeux qui prélève la substance de l'espace de prélèvement (29) et l'évacue à partir de cet espace dans au moins une ouverture d'entrée (36), déterminant une direction d'écoulement, d'un canal de sortie (21), caractérisé en ce que l'embouchure du flux gazeux (35) est orientée à peu près en sens contraire à la direction d'écoulement de l'ouverture d'entrée (36).

2. Distributeur selon la revendication 1, caractérisé en ce que la source de gaz sous pression comporte au moins une pompe (9) possédant une chambre de pompage (10) variable manuellement, en particulier une pompe à piston de refoulement se raccordant axialement directement à un réservoir de substance (4) et possédant un piston de pompage (13), dont la chambre de pompe (10) présente de préférence une largeur au moins égale à la largeur du réservoir de substance (4) et/ou de la partie restante du distributeur (1).

3. Distributeur selon la revendication 1 ou 2, caractérisé en ce qu'au moins un canal d'écoulement (18) de la source de gaz sous pression (9) traverse un réservoir de substance (4), en particulier pour l'essentiel coaxialement, et/ou que le réservoir de substance (4) est réalisé sous une forme essentiellement annulaire en section droite.

4. Distributeur selon une des revendications précédentes, caractérisé en ce que la source de gaz sous pression (9) est raccordée à travers au moins un injecteur (37) pour l'entraînement de la substance, ou à travers au moins une chambre de turbulence (38) pour la substance, à une ouverture de sortie (24), au moins une embouchure de flux gazeux (36) débouchant de préférence comme un éjecteur (35) dans la chambre de turbulence (38), et/ou qu'au moins un injecteur (37) est prévu dans la zone du début d'un canal de sortie (21), en particulier d'un canal de sortie ascendant.

5. Distributeur selon une des revendications précédentes, caractérisé en ce qu'un canal d'écoulement (18) est pourvu d'une ouverture d'entrée de substance répartie sur sa périphérie, ouverture qui est de préférence précédée d'une soupape commandée (40) pour le canal d'écoulement (18) et est réalisée en particulier comme une perforation d'une enveloppe de canal séparant le canal d'écoulement et la chambre à substance.

6. Distributeur selon une des revendications précédentes, caractérisé en ce qu'une surface de fond (29) est prévue comme une aire de prélèvement de l'espace de prélèvement à remplir à partir de la chambre à substance (4), surface vers laquelle est dirigée de préférence l'embouchure de flux gazeux (35) prévue comme embouchure de gaz sous pression et en face de laquelle est située, à distance, l'ouverture d'entrée (36) du canal de sortie (21), l'ouverture (36) étant de préférence entourée en anneau par l'embouchure de flux gazeux (35).

7. Distributeur selon une des revendications précédentes, caractérisé en ce qu'il comprend un dispositif de dosage (25) pour la substance, qui est pourvu de préférence d'une chambre de dosage (26) présentant deux parties de chambre (27, 28) déplaçables l'une par rapport l'autre entre une position de remplissage et une position de vidage, le dispositif de dosage étant agencé en particulier dans la zone du fond de la chambre à substance (4), et que, de préférence, une partie de chambre inférieure (27) est réalisée de préférence en forme de coupelle et délimitée en particulier, au bord, par un entonnoir de déversement (30), et qu'une partie de chambre supérieure (28) est formée en particulier par une partie d'enveloppe extérieure d'un canal d'écoulement de gaz sous pression (18), ayant une forme annulaire en section ou entourant un canal de sortie (21), ladite partie d'enveloppe pouvant être engagée en particulier, avec ajustement, dans une paroi latérale de la partie de chambre inférieure (27), la chambre de dosage (26) pouvant être fermée de préférence par une commande, en particulier en fonction de la course ou de la pression, avec du retard par rapport au refoulement de gaz sous pression, et une partie de chambre mobile (28) étant disposée en particulier sur un piston exposé au gaz sous pression, par exemple sur le piston de pompage (13).

8. Distributeur selon une des revendications précédentes, caractérisé en ce qu'une partie de chambre mobile (28) de la chambre à substance (4) traverse, à une extrémité située à l'opposé d'une autre partie de chambre (27), une paroi extrême (6) coordonnée, et est de préférence montée mobile, à peu près axialement, à l'encontre d'une force élastique, à l'aide d'une bague retournée à double paroi (32), la partie de chambre mobile (28) étant sollicitée en particulier, suivant des directions contraires, par des ressorts (33, 34) de puissances différentes.

9. Distributeur selon une des revendications précédentes, caractérisé en ce que, pour le transport de la substance, seulement une pompe (9) est prévue, dont le piston de pompage (13) et/ou le cylindre de pompe est disposé ou monté par au moins un assemblage à encliquetage (14, 15) à la chambre à substance (4).

10. Distributeur selon une des revendications précédentes, caractérisé en ce qu'un canal d'écoulement de la source de gaz sous pression (9), en particulier le canal de sortie (21), est formé par un canal télescopique qui traverse de préférence la chambre de pompe (10) et/ou est divisé en parties de canal (22, 23) dont l'une est disposée sur un piston de pompage (13) et une autre partie de canal est disposée sur l'un de deux éléments d'actionnement (41, 42) déplaçables l'un par rapport à l'autre et formés par des parois extrêmes éloignées l'une de l'autre de la chambre à substance (4) et de la chambre de pompe (10).
